# EUROPEAN PATENT APPLICATION

(11) **EP 3 254 615 A1**
(43) Date of publication of application: **13.12.2017**
(21) Application number: 17180681.3
(22) Date of filing: 30.08.2013
(51) Int. Cl.: A61B 5/00, A61B 8/00, A61B 7/04, A61B 7/00, A61B 5/0488, A61B 5/053, A61B 5/08, A61B 5/113, A61B 5/107

(54) **MULTISENSOR WIRELESS ABDOMINAL MONITORING APPARATUS, SYSTEMS, AND METHODS**

(30) Priority: 07.09.2012 US 201261697951 P
(62) Divisional of application: 13836008.6
(71) Applicant: The Regents of the University of California, Oakland, CA 94607-5200 (US); The United States Government as represented by The Department of Veterans Affairs, Washington, DC 20420 (US)
(72) Inventor: SPIEGEL, Brennan, Los Angeles, California 90048 (US); KAISER, William, Los Angeles, California 90095 (US)
(74) Representative: Richards, John

(57) **Abstract**

A multi-sensor wireless abdominal monitoring system comprising a low-profile belt that fits around the abdomen and is embedded with specialized wireless sensors. The system is configured to continuously monitor a range of gastrointestinal and abdominal wall functions. The system wirelessly transmits data to an external device, such as a smartphone or computer for storage and download to a central server. The acquired data may be monitored remotely through specialized software that generates clinically interpretable information presented through a graphical user interface. The device provides data that are immediately actionable for a wide range of inpatients and outpatients with high prevalence disorders in varied clinical settings.

## Description

### CROSS-REFERENCE TO RELATED APPLICATIONS

This application is a nonprovisional of U.S. provisional patent application serial number 61/697,951 filed on September 7, 2012, incorporated herein by reference in its entirety.

### STATEMENT REGARDING FEDERALLY SPONSORED

### RESEARCH OR DEVELOPMENT

This work was supported by the U.S. Department of Veterans Affairs, and the Federal Government has certain rights in this invention.

### INCORPORATION-BY-REFERENCE OF MATERIAL

### SUBMITTED ON A COMPACT DISC

Not Applicable

### NOTICE OF MATERIAL SUBJECT TO COPYRIGHT PROTECTION

A portion of the material in this patent document is subject to copyright protection under the copyright laws of the United States and of other countries. The owner of the copyright rights has no objection to the facsimile reproduction by anyone of the patent document or the patent disclosure, as it appears in the United States Patent and Trademark Office publicly available file or records, but otherwise reserves all copyright rights whatsoever. The copyright owner does not hereby waive any of its rights to have this patent document maintained in secrecy, including without limitation its rights pursuant to 37 C.F.R. § 1.14.

### BACKGROUND OF THE INVENTION

### 1. Field of the Invention

This invention pertains generally to abdominal monitoring, and more particularly to monitoring for abdominal dysfunctions.

### 2. Description of Related Art

To our knowledge, there are currently no commercially-available systems that provide continuous, wireless, abdominal physiologic monitoring across the spectrum of abdominal dysfunctions that we describe herein. The "state of the art" includes conducting intermittent abdominal examinations with a stethoscope and manual palpation.

### BRIEF SUMMARY OF THE INVENTION

One aspect of the present invention is a multi-sensor wireless abdominal monitoring system comprising a low-profile belt that fits around the abdomen and is embedded with specialized wireless sensors. The system is configured to continuously monitor a range of gastrointestinal and abdominal wall functions. The system wirelessly transmits data to an external device, such as a smartphone or computer for storage and download to a central server. The acquired data may be monitored remotely through specialized software that generates clinically interpretable information presented through a graphical user interface. The device provides data that are immediately actionable for a wide range of inpatients and outpatients with high prevalence disorders in varied clinical settings, discussed below.

The system and methods of the present invention accomplish some or all of these functions: (1) real-time acoustic monitoring to measure the continuous sounds of motility emanating from the gastrointestinal tract and to measure heart rate by monitoring abdominal arterial pulse; (2) abdominal girth monitoring to measure static and dynamic changes in abdominal circumference over time; (3) electromyography to measure contractions of the anterior abdominal musculature; (4) motion sensing to infer breathing rate and other forms of movement; (5) galvanic skin conductance to infer emotional or physical stress; and (6) global positioning to track movement and location.

Another aspect of the present invention is a multi-sensor abdominal monitoring system incorporating a low-profile belt that fits externally around the abdomen and is embedded with specialized wireless sensors.

In one embodiment, the system is configured to continuously, safely, and comfortably monitor intra-abdominal acoustic signals and abdominal girth, and stores the data in a HIPAA compliant hard drive.

In another embodiment, the system is configured to wirelessly transmit data to a smartphone for storage and download to a central server. The data from AbStats are monitored by specialized software that will generate clinically interpretable information presented through a graphical user interface. The system is configured to provide data that are immediately actionable for a wide range of inpatients and outpatients with high prevalence disorders in varied clinical settings.

In another embodiment, the system provides real-time acoustic monitoring to measure the continuous sounds of motility emanating from the gastrointestinal tract. In addition, it may be configured to measure abdominal girth to monitor static and dynamic changes in abdominal circumference over time.

As discussed in detail, below, the present invention provides a novel tool for patients and providers to track disease between scheduled visits, and offers the general public a way to quantify their own physiology.

Further aspects of the invention will be brought out in the following portions of the specification, wherein the detailed description is for the purpose of fully disclosing preferred embodiments of the invention without placing limitations thereon.

### BRIEF DESCRIPTION OF THE SEVERAL VIEWS OF THE DRAWING(S)

The invention will be more fully understood by reference to the following drawings which are for illustrative purposes only:
FIG. 1 is a top schematic view of a multi-sensor wireless abdominal monitoring system in accordance with the present invention.
FIG. 2 is a schematic side view of the waist belt and digital stethoscope-type transducer of FIG. 1.
FIG. 3 is a schematic diagram illustrating an exemplary array of apertures configured to receive individual sensors in accordance with the present invention.
FIG. 4A is a schematic top view of an abdominal girth sensor system in accordance with the present invention that comprises a compliant belt that may be worn around the abdomen of a user.
FIG. 4B is a schematic diagram that shows the sensor system of FIG. 4A with an abdominal expansion of the belt.
FIG. 5 is a schematic diagram for a system architecture for the wireless abdominal monitoring systems shown in FIG. 1 through FIG. 4B.
FIG. 6 is a flow diagram for a sensor fusion process in accordance with the present invention.

### DETAILED DESCRIPTION OF THE INVENTION

### A. System Description

### 1. Acoustic Sensor Belt and System

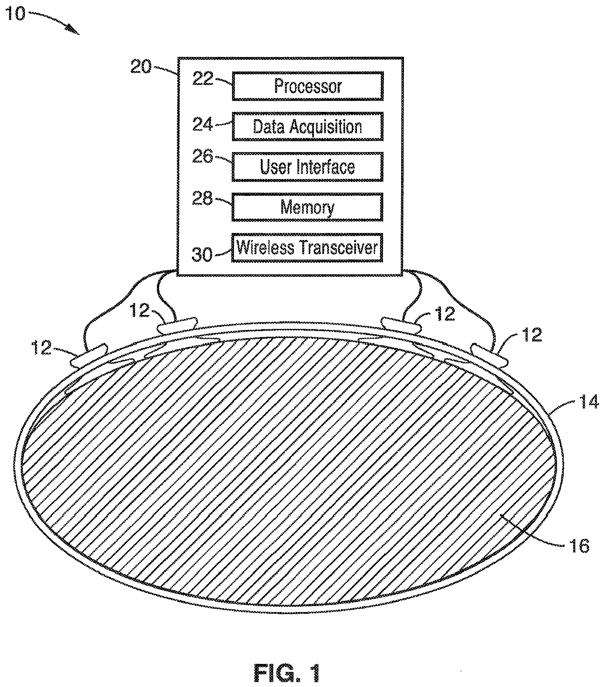

Referring to FIG. 1, one embodiment of the multi-sensor wireless abdominal monitoring system 10 comprises a plurality of low profile highly focused acoustic sensors 12 that are applied to the anterior abdominal wall 16, e.g. to perform acoustic monitoring to measure the sounds of motility emanating from the gastrointestinal tract and to measure heart rate by monitoring abdominal arterial pulse.

FIG. 1 illustrates a system 10 with a four-sensor linear array of digital stethoscope transducers 12 according to a preferred embodiment of the invention. The sensors 12 are supported by an elastic waist belt 14 that is configured to circumscribe the patient's abdomen 16.

It will be appreciated that other sensor configurations are possible as needed for multiple-sensor scenarios. In case of a 2-sensor system, with one sensor positioned on waist belt 16 such that it lines up adjacent to the right lower quadrant of the abdomen 16 over the region of the ileocecal valve (not shown), and the other sensor 12 lining up adjacent the left lower quadrant of the abdomen 16 over the sigmoid colon (not shown).

These acoustic sensors 12 are configured to continuously and non-invasively monitor and capture a range of audio signals which represent gastrointestinal and abdominal wall functions. The captured data is recorded by the system 10 in a synchronized manner from all onboard sensors 12. The data acquired by the sensors 12 are fed to a control unit 20 housing signal processing and data acquisition components. A data acquisition unit 24 and processor 22 acquire the raw data from the sensors 12. The unit may have a user interface 26 for user manipulation of the device, and memory 28 for storing the acquired data and/or programming associated with data acquisition/signal processing. A wireless transceiver 30 may also be included for transferring the acquired sensor data and/or configuration data to a central repository or database (not shown).

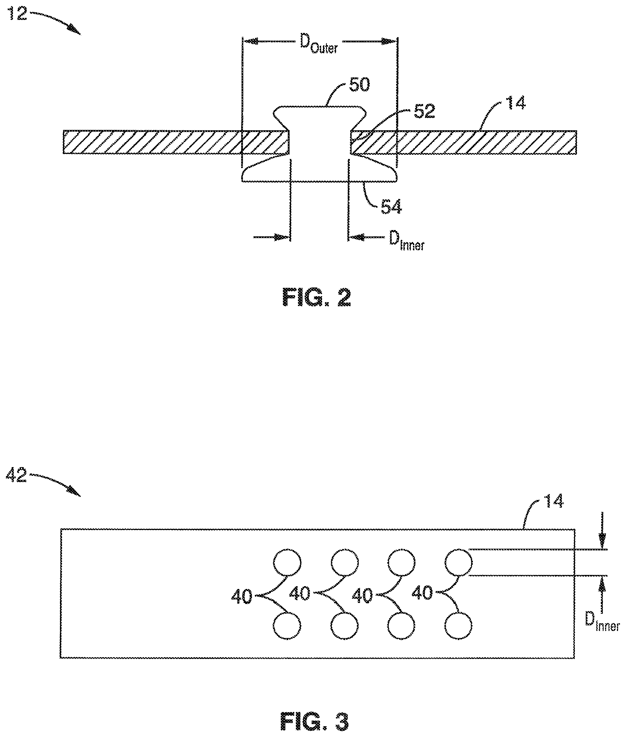

FIG. 2 shows a schematic side view of the waist belt and digital stethoscope-type transducer 12. The sensor has a sensing face 54 with a diameter Dₒᵤₜₑᵣ larger than the waist section 52 diameter Dᵢₙₙₑᵣ and top portion 50. Thus, the sensor 12 is supported within an aperture 40 (see FIG. 3) of the belt 14.

FIG. 3 shows an exemplary array 42 of apertures 40 configured to received individual sensors. Each aperture 40 is preferably sized to have a diameter matching the diameter Dᵢₙₙₑᵣ of the sensor 12 waist section 52. It is appreciated that array 42 may be configured with any number or pattern of apertures 40.

While sensors 12 shown in FIG. 1 through FIG. 3 are illustrated with respect to one or more acoustic sensors for monitoring and capturing a range of audio signals which represent gastrointestinal and abdominal wall functions, it is also appreciated that other types of sensors may be employed in combination with or in alternative to, said acoustic sensors. For example, sensors 12 may comprise one or more of: capacitance measuring electrodes for abdominal girth monitoring to measure static and dynamic changes in abdominal circumference over time; electromyography sensors to measure contractions of the anterior abdominal musculature; motion sensors to infer breathing rate and other forms of movement; galvanic skin conductance sensors to infer emotional or physical stress; and global positioning sensors to track movement and location.

### 2. Abdominal Girth Sensor

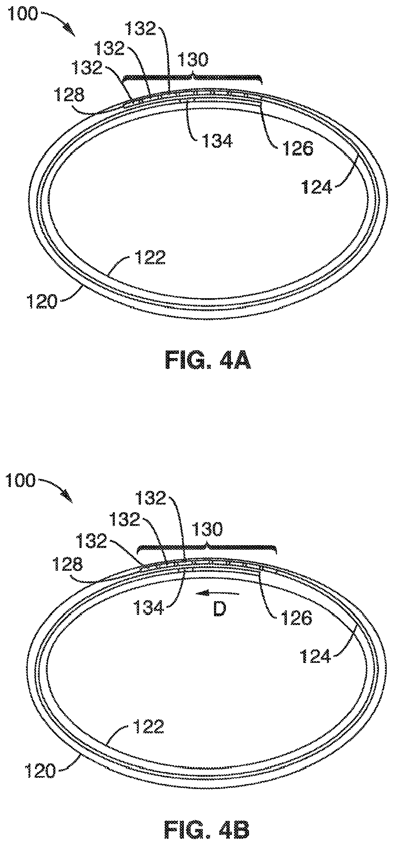

FIG. 4A and FIG. 4B show top views of an abdominal girth sensor system 100 in accordance with the present invention that comprises a compliant belt that may be worn at the abdomen 16 of a user. System 100 is configured for both wearable, convenient usage as well as support of other sensors. In addition, the girth sensor 100 can be integrated with other garments and systems that assist clinical usage as well.

System 100 uses an outer elastic housing 120 and inner elastic housing 122 that entrain an inelastic inner belt 124. The elastic characteristics of belt 100 may be adjusted by selection of materials (for example Lycra or other elastic fabric for housings 120 and 122). The inelastic belt 124 is preferably composed of a mechanically non-compliant material (e.g. nylon cloth or the like fabric), which is attached to the outer elastic housing 120 and inner elastic housing 122 at its the ends 126, 128.

The inner belt 124 is equipped with a flexible, conducting fabric electrode array 130. At one end 126 of the inner inelastic belt 124 is a single inner capacitor electrode 134, and at its opposite end 128 is the outer linear capacitor electrode array 130. While eight electrodes 132 are shown to comprise the array 130 in FIG. 4A and FIG. 4B, it is appreciated that system 100 can be implemented with many more electrodes 132, e.g. preferably over 64 electrodes 132.

Integrated capacitance detection elements 132, 134 of the inner belt 124 are configured to detect relative motion and provide an absolute circumference and girth measurement. FIG. 4A shows the belt 100 in an initial configuration. FIG. 4B shows the belt 100 after an expansion of the abdomen, resulting in free end 126 and sensor 134 moving distance D with respect to the array 130.

In a preferred embodiment, the electrodes 132 are addressed by controller (e.g. see controller 20 of FIG. 1) that comprises an analog multiplexor component and a standard capacitance detection circuit (both not shown) to enable access to each electrode 132. As shown in FIG. 4A and 4B, when girth expansion occurs, a relative displacement D occurs at the ends 126 and 128 of the inelastic belt 124. This displacement D changes the position of the electrode 134 with respect to electrodes 132 in array 130. Since measured capacitance depends on the location of the inner 134 and outer electrodes 132, then displacement and girth can therefore be measured.

### 3. Monitoring System Architecture

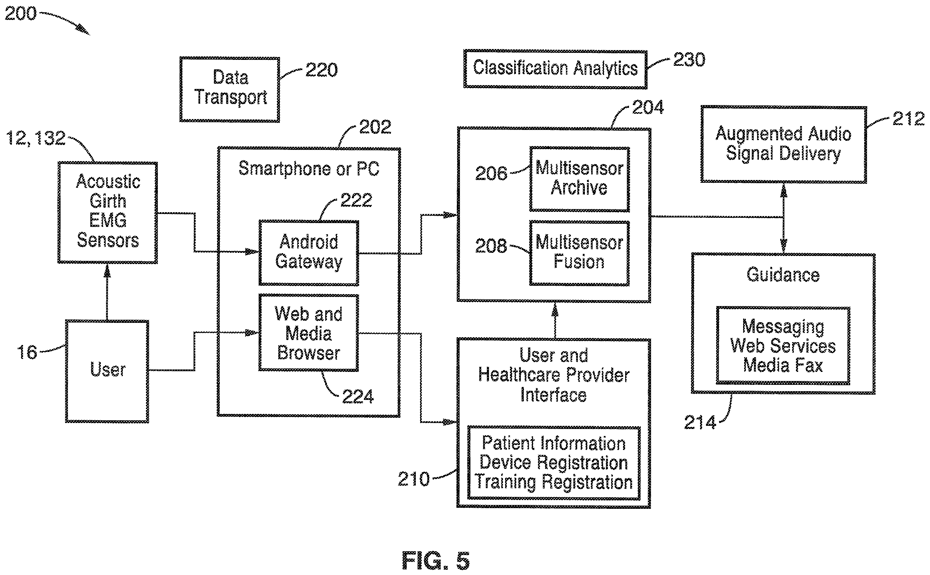

FIG. 5 shows a preferred embodiment for a system architecture 200 for the wireless abdominal monitoring systems 10, 100 shown in FIG. 1 through FIG. 4B. System architecture 200 uses a sensor fusion module 204 for the identification, classification, and notification of events and conditions related to digestive and abdominal health. System architecture 200 links multiple sensors (e.g. sensors 12 or 132 in FIG. 1 through FIG. 4B) at the user 16 and provides classification analytics 230 capabilities at the sensor fusion module 204, and at the local wearable gateway 20, or via data transport 220 to a smartphone (e.g. Android gateway 222 for receiving data from sensors 12, 132) or PC 202 with web and media browser 224 for user 16 access. Sensor fusion module 204 combines data from multiple acoustic sensors 208 as well as an archive 206 diverse sensor systems including those of abdominal girth, electromyography, and others.

A web services module 214 may be included to provide guidance through services such as messaging, media, fax, etc. User and healthcare provider support module 210 provides data analysis modules to access the data and provide extended functionality such as alerts, compliance mechanisms, patient information, device and training registration, etc.

The system 200 allows for the tracking of patient usage compliance, patient measurement trend analyses, user and device management, and provides a method for improving compliance in gastrointestinal healthcare.

System 200 may also include an audio component 212 for providing analytics within an augmented audio signal delivery interface. This provides remote access to human audible signals as acquired at the patient 16. A clinician, trained technician, or the user may be provided with this source that itself may include processing for reduction of noise and enhancement of desire features. The augmented audio signal interface 212 may employ standard methods for removal of noise and / or echo sources, or commercially available audio signal processing.

Monitoring system architecture 200 is illustrated in FIG. 5 with the user (patient) 16 at left and equipped with one or more of the sensor systems 12, 132 that emphasize acoustic sensing, but may also include electromyography and many others. Sensor data is preferably transported via data transport 220 (which may include transceiver 30) to a smartphone or other computing platform 203 where a gateway system (e.g. Android software implementation 22) provides data transport to the classification analytics system 230. The same smartphone or other computing platform 202 may also provide the user with access to data by methods that may include but are not limited to web user interfaces 224. Data arriving at the classification analytics system 230 may then be processed by methods including sensor fusion module 204. Then data may be made available to healthcare providers and partners via module 210. At the same time, clinicians and others may be presented with audible sound sources from module 212 via remote access (including telephony and Web based methods) for interpretation. Further guidance information derived from services module 214 may also be provided to the user 16.

### 4. Data Flow and Analytics

The systems 10, 100 acquire data via acoustic sensing at the subject 16. Multiple sensors (e.g. 12, 132) are generally applied at multiple abdominal locations. Acquired data may be processed at the local wearable computing gateway 20 or transported via wireless and/or Internet services to smartphone/PC 202 or analytics server 230 shown in FIG. 5. The system 10, 100 may also store data locally within memory 28 at the wearable gateway 20, enabling operation independent of network interfaces.

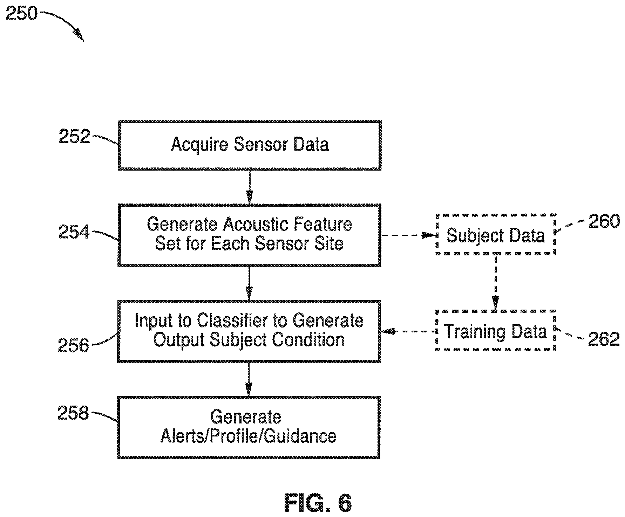

An integral component of the analytics functionality of system 200 is sensor fusion module 204. FIG. 6 shows sensor fusion process 250 which acquires sensor data at step 252 to generate an acoustic feature set for each sensor site via frequency and time domain signal processing at step 254. Bayesian sensor fusion/classification is then applied at step 256 to producing an inference regarding subject state/condition. Step 256 may also include as input subject data 260 and training data 262 based on a library of signals obtained in trials on subjects of known state. Additional sources of evidence, including abdominal girth and other clinical measures, may also be included.

In the feature set step 254, acoustic or other signal sources are processed to extract features that may include but are not limited to:
amplitude, primary frequency components, time of occurrence of signals and coincidence of signals. The features from step 254 are then applied as an input to a classifier as step 256, which may include, but is not limited to, a selection of Bayesian classifiers. As mentioned above, the Bayesian classifier produces an output computation corresponding to an inference for the subject condition at step 356. Preparation of the Bayesian classifier by training process steps 260, 262 is based on the presentation of feature sets for corresponding patient condition and for many patient conditions. Through this training process, the Bayesian classifier 256 acquires the capability to infer the actual subject state from knowledge only of features. Data from multiple sensors 12, 132 may be acquired at step 252 by a time-synchronized sampling method and then stored and made available in either real time or at a later time to the computational system 200 supporting sensor fusion.

The analytics is then followed by guidance at step 258 delivered to users and caregivers. These include alerts regarding change in subject condition, time profiles of specific subject states, guidance for users and caregivers, and information regarding trends.

### B. Clinical Applications

Systems 10, 100, 200 and method 250 may be useful in a broad spectrum of patients and indications. Exemplary indications may be found in Table 1 below, and include:
1. Monitoring-patients with post-operative ileus. Most every patient develops temporary bowel paralysis following thoracic, abdominal, or pelvic surgeries. Clinicians monitor stool output and bowel sounds to determine when it is safe to begin re-feeding and to plan hospital discharge. Patients with prolonged bowel paralysis can require additional hospital days and excessive stays. With system 10 and 200, patients may be monitored continuously, and data be made available on a web-based dashboard (e.g. web interface 210) for real-time evaluation. After establishing normative and predictive standards for our invention, providers will more reliably determine who is recovering quickly based on acoustic and abdominal girth monitoring, and who may require additional close observation. For example, patients with early evidence of motility and resolving abdominal distension, detected by our invention, acoustic monitoring and abdominal circumference tracking, may be fed earlier and discharged sooner than might otherwise occur without use of our invention.
2. Monitoring in-patients with an "acute abdomen." Patients with an acute abdomen are highly prevalent in medical, surgical, pediatric, and obstetrical inpatient wards, and include those with acute cholecystitis, pancreatitis, appendicitis, colitis, diverticulitis, bowel ulcerations or perforations, and bowel obstructions, among many other conditions. These high acuity patients often have a dynamic and unpredictable clinical course and require frequent monitoring. Although acutely ill patients often have continuous cardiovascular and pulmonary monitoring, they do not have continuous abdominal monitoring. Yet, changes in the abdomen almost always precede changes in cardiovascular and pulmonary function in patients with acute abdominal disorders. Our invention will provide real-time data on a range of functions in acutely ill Gl patients. For example, patients with progressive disease often exhibit "peritoneal signs" marked by abdominal wall contractions from pain, detectable through electromyography. They may develop abdominal distension in the setting of progressive ileus or bowel obstruction, monitored continuously through abdominal girth measurements. They may develop intermittent high-pitched bowel sounds from obstruction, measured continuously through acoustic monitoring. Furthermore, they may develop other evidence of distress, marked by a combination of increased skin conductance, heart rate, and respiratory rate - all measurable through the use of the systems 10, 100 of the present invention even without concurrent cardiopulmonary monitoring. The systems 10, 100 are invaluable to predict clinical decrement earlier than otherwise possible. Conversely, the systems 10, 100 may reveal signs of clinical stabilization in ways not easily detected through usual clinical approaches, which rely upon intermittent check-ups throughout the day - not continuous data monitoring of abdominal physiology. A 30 second abdominal examination cannot provide reliable data about what has occurred, or will occur, in the course of a day - it can only provide data about the immediate moment in time. Thus, there is an extensive need for continuous abdominal physiological monitoring, especially in patients with acute abdominal processes.
3. Monitoring inpatients in intensive care units. Even in the absence of an "acute abdomen," abdominal monitoring is standard of care in all patients, especially those in intensive care units. Of note, this is especially important for patients who cannot easily verbalize their abdominal symptoms, regardless of abdominal acuity, including patients who are demented, delirious, comatose, sedated, intubated, or infants or children with limited verbal capacity. In addition, patients in intensive care often receive narcotics for pain control, which themselves lead to diminished bowel motility and risk of ileus or Ogilve's syndrome (colonic inertia). Patients in intensive care may also develop intra-abdominal infections or inflammatory conditions that are easily missed in their early phase. The systems 10, 100 of the present invention accurately detect these conditions earlier than usual by demonstrating increased abdominal wall contractions - a sign of abdominal pain. The systems 10, 100 are also configured to monitor bowel sounds as a screen for progressive ileus from medications or intra-abdominal processes, and detect early signs of bowel obstruction through monitoring abdominal circumference, coupled with acoustic and electromyographic data.
4. Monitoring outpatients with ascites. Cirrhosis is highly prevalent, and ascites (abdominal fluid accumulation) is a common complication of portal hypertension in chronic liver disease. Ascites also occurs in a range of other conditions, including abdominal infections and cancer. Patients with ascites can be difficult to manage and require frequent changes in their diuretic (water pill) dosing, amount of sodium restriction, and dietary composition. The systems 10, 100 of the present invention provide a novel and actionable way to monitor ascites in outpatients with cirrhosis. In patients with recurrent or recalcitrant ascites, continuous monitoring with the systems 10, 100 provide deep insight into the progression and timing of fluid accumulation within the abdomen. The systems 10, 100 can detect worsening of ascites by remotely reporting evidence of abdominal distension and diminished amplitude of bowel acoustics, suggesting increased distance between the sensor and intestinal track from interposed fluid accumulation. The systems 10, 100 can alert healthcare providers if there is a marked change in abdominal circumference within set parameters, allowing providers to intervene long before the patient might otherwise be scheduled for the next clinic visit. Additionally, the data can be reviewed over a several week course to evaluate for trends. For example, the "electronic ascites journal" provided by our invention might reveal consistent worsening on Mondays. Upon further evaluation, the patient might admit to salt-heavy dinners on Sunday nights. The clinical plan would be clear: either cutback on the salty dinners on Sunday night, or increase the dose of diuretics on Sunday in advance of Monday's clinical fall-out. Without the systems 10, 100 of the present invention, this discussion would probably never occur.
5. Monitoring inpatients and outpatients receiving narcotics for acute or chronic pain. Narcotics are commonly used in healthcare. Patients with chronic pain, in particular, are highly prevalent in both the inpatient and outpatient settings. A major complication of narcotics is bowel paralysis, leading to ileus or even Ogilve's syndrome (colonic inertia). For inpatients, bowel paralysis can lead to prolonged stays and increased healthcare costs. For outpatients, bowel paralysis can impact compliance with otherwise effective therapies. For both groups, narcotics cause disruptive consequences of Gl distress, and ultimately diminished health related quality of life. Other than following patient symptoms in a reactive manner, the systems 10, 100 allow proactive management of all patients on narcotics, especially those just beginning narcotics and those struggling to find the correct dose. With systems 10, 100, providers can monitor the cause-and-effect consequences of narcotics, and employ these data to optimize the correct dose and timing. For example, patients with progressive signs of bowel paralysis and abdominal distension should have their dose reduced to compensate. Patients with persistent pain and normal bowel function might require higher or more frequent therapeutic dosing. In short, systems 10, 100 provide a new paradigm in how to manage and optimize the care of patients on narcotics.
6. Monitoring outpatients with inflammatory bowel disease (IBD). Inflammatory bowel diseases, including Crohn's disease and ulcerative colitis, are common conditions in Gl practice. Patients with Crohn's Disease, in particular, often suffer from intermittent bowel obstructions related to intestinal strictures. Managing these patients can be challenging and not well suited to the usual approach of intermittent appointments and clinic visits. The disease can be dynamic, and bowel obstructions may occur between clinic visits, leading patients to the emergency department for urgent management. With wireless monitoring provided by systems 10, 100, healthcare providers can monitor their IBD patients in real-time, and identify early signs of bowel obstruction remotely, accurately, and quickly. For example, the systems 10, 100 might reveal evidence of increasing frequency of high amplitude bowel sounds over the course of several days, along with increased frequency of abdominal wall contractions, increases in heart rate, galvanic skin resistance, and respiratory rate. Patients themselves may detect these early changes as well, but decide to wait for their scheduled visit, or opt to wait until the symptoms pass. However, providers may interpret the data differently, allowing them to proactively contact their patient, potentially modify therapy, or schedule an earlier appointment. If coupled with patient reported symptoms entered through the system, the marriage of informatics and patient reporting would enrich the data even further.
7. Diagnosis of outpatients with unexplained abdominal pain. Chronic or recurrent abdominal pain is highly common both in Gl and general medicine clinics. Although various imaging tests are commonly used to evaluate the source of pain (e.g. computerized tomography, magnetic resonance, etc.), there are very few functional tests of abdominal physiology. Most tests of abdominal function (e.g. esophageal or gastric motility, "Smartpill," Sitzmark studies, etc.) are highly specialized, invasive, expensive, and limited to specialized motility centers. Moreover, these tests only measure health over a short period of time, typically much less than a day. In contrast, systems 10, 100 provide continuous physiologic data over prolonged periods. When coupled with a diary of patient reporting, systems 10, 100 provide concurrent data to better understand the underlying physiology leading up to, during, and after pain flares.
8. Diagnosis of outpatients with abdominal bloating. Bloating is among the most common symptom experienced by people in the community, and certainly in Gl practices. Despite being common, little is known about its pathophysiology, and even less is available to manage the condition. However, there are ways to manage bloating if they are aligned with the underlying physiology. For example, most patients with bloating have a learned reflex where the anterior abdominal wall is actively relaxed, forcing the abdomen to literally "pooch out" with lowering of the diaphragm. This form of "functional bloating" is different from increased intra-abdominal gas, where the abdominal wall does not relax. By measuring abdominal circumference with abdominal electromyography and bowel acoustics, providers can better establish whether bloating is from abnormal gas handling, abnormal motility, or abdominal wall relaxation. The treatment approach is different for each type of bloating (as different as behavioral modifications versus antibiotics). Absent the systems 10, 100 of the present invention, there is currently no way to monitor bloating outside of specialized protocols run over short time periods.
9. Diagnosis of outpatients with nausea and vomiting. Nausea and vomiting are highly disruptive symptoms. Although the underlying etiology is often apparent from diagnostic testing, many patients continue to have unexplained nausea and vomiting despite costly and extensive work-ups. The systems 10, 100 provide another tool to help understand the sequence of events surrounding bouts of nausea and vomiting. When placed in the epigastrium, the systems 10, 100 measure gastric acoustics and infer the frequency and amplitude of gastric motility - especially relevant in gastroparesis, such as in patients with advanced diabetes. The systems 10, 100 further provide information on the sequence of key physiologic events, including motility, abdominal contractions, and stress, among other functions. The systems 10, 100 will also detect when a patients is likely eating, and how far between meals the nausea and vomiting occur. It is expected that systems 10, 100 will reveal new and previously unknown physiologic patterns and sequences related to otherwise unexplained nausea and vomiting.
10. Diagnosis of outpatients with diarrhea and/or fecal incontinence. Patients with diarrhea and incontinence may suffer from many different conditions with different physiologic mechanisms. Patients with fecal incontinence, in particular, can be difficult to diagnose and treat. The systems 10, 100 provide another tool to help understand reasons for diarrhea and incontinence. For example, if systems 10, 100 detected a mass colonic movement in advance of fecal incontinence, it would suggest the otherwise normal anorectal function was overwhelmed by volume, and argue against intrinsic dysfunction of anorectal mechanisms. In contrast, if mass movements did not precede fecal incontinence, then it would suggest that anorectal dysfunction was the most likely explanation. In addition, systems 10, 100 can track motility in relation to meals, stress, and other physiologic events. The data might reveal patterns that are explanatory and clinically actionable.
11. Diagnosis of outpatients with constipation. Constipation is also extremely common. Gastroenterologists divide constipation into 3 major forms: (1) slow transit, (2) normal transit, and (3) pelvic dyssynergia. There are invasive tests to help separate these forms of constipation, but they are expensive, only available in specialized centers, and provide only short-term data. The systems 10, 100 provide another tool to better understand the underlying mechanisms of constipation. For example, patients with constipation despite evidence of colonic peristalsis likely have either "normal transit" or pelvic dyssynergia causing their constipation. In contrast, lack of peristalsis would suggest "slow transit" constipation. The treatments are very different.
12. Monitoring and further diagnosis of outpatients with irritable bowel syndrome (IBS). IBS affects ∼10% of the world's population, and is among the most common conditions experienced by man. Marked by abdominal pain and defecatory symptoms, IBS is not only common, but also a significant impact on the quality of life. Current theories of IBS suggest an abnormality in the "brain-gut axis," with both central and peripheral disease mechanisms. The "stress" theory of IBS suggests that Gl symptoms arise from visceral anxiety driven through a combination of stress hormones and their function on Gl physiology. Peripheral theories include abnormal motility, inflammatory changes in the intestines, and bacteria, among others. Patients with IBS have recurrent symptoms that can be dynamic and unpredictable. It is difficult to monitor the condition correctly, yet diagnostic and therapeutic decision making depends on valid and reliable patient reporting of their illness. The systems 10, 100 may revolutionize how we monitor and categorize patients with IBS. For example, patients with predominantly "central" mechanisms of IBS might develop stress initially, and Gl physiologic abnormalities secondarily. These patients might develop changes in galvanic skin resistance, heart rate variability, and/or tachypnea in the minutes, hours, or even days before an "IBS flare." This temporal relationship might indicate that stress is driving the Gl symptoms, and not the other way around. In contrast, patients might first develop high-pitched bowel sounds, evidence of colonic mass movements, and diarrhea (itself evidenced through Valsalva maneuvers on electromyography, stable positioning in a known bathroom on GPS, and characteristics acoustic signals), and only then develop changes in galvanic skin resistance. This might suggest that peripheral mechanisms are predominant.
13. Serve as a bowel diary. Regardless of the underlying condition, Gl patients often need to keep an accurate bowel diary to help monitor disease, treatments, and collect diagnostic information for their providers. But bowel diaries are frequently inaccurate and patients often forget to complete the diary or even fail to begin. The systems 10, 100 provide an objective way to monitor bowel movement frequency and duration. Through electromyography, global positioning, and acoustic monitoring, our invention will infer when bowel movements are occurring. If coupled with other information, including dietary intake and composition, medicine intake and timing, and other external factors, systems 10, 100 provide insight about the timing and potential causes of constipation and diarrhea.
14. Monitoring patients with high-risk abdominal aortic aneurysms (AAA). In the setting of cardiovascular diseases, such as hypertension, smoking, and hyperlipidemia, the abdominal aorta can develop atherosclerotic changes. Over time, this leads to aneurysm formation, itself potentially dangerous; aortic hemorrhage can be rapidly fatal. Patients with known "triple As" (AAA) are typically monitored with serial ultrasounds. But AAAs can change rapidly between scheduled ultrasounds. Continuous acoustic monitoring can listen to the continuous "hum" of an AAA, and track changes in the acoustic signature. Marked changes might indicate expansion or other morphological changes of the lesion, and therefore prompt more timely investigation before a potential catastrophe. The systems 10, 100 can be placed directly over an AAA in the supra-umbilical midline and continuously monitor and characterize the acoustic signature of an AAA.
15. Monitoring infants with colic. Colic is common and troubling for infants and their parents. Colic can lead to abdominal pain and distress for the whole family. The systems 10, 100 can be used to provide better information about what is happening inside a colicky infant, identify early signs of colic, and provide a head start for parents to sooth or otherwise manage their infant prior to the peak of colic attacks.
16. Monitoring infants or young children with Gl disorders. Beyond colic, infants and children may develop a range of Gl illnesses. Unlike adults, infants or children cannot reliably transmit their pain or distress. The systems 10, 100 may be used to provide early and objective signs of distress, including abdominal contractions, increased heart and breathing rates, galvanic skin resistance changes, and increased amplitude contractions. This can warn parents of impending or ongoing pain and help achieve more timely and effective disease-targeted therapies.
17. Monitoring nursing home patients with diminished verbal ability. Similar to monitoring infants or young children, many nursing home patients have lost their ability to verbalize symptoms, especially pain. The systems 10, 100 can be used to provide objective, real-time data to suggest possible abdominal pain.
18. Alerting caretakers to bowel movements in infants and toddlers. Caretakers often guess whether their infant or toddler has passed a bowel movement. The systems 10, 100 can be used to provide objective evidence when it occurs, and alert caretakers to check the diapers.
19. Provide objective endpoints for clinical trials. There are few reliable and objective markers in Gl clinical trials, especially for trials of functional Gl disorders like IBS, or mal-digestive conditions like lactose intolerance. The FDA is focused on identifying valid and reliable markers for trials. The systems 10, 100 can be used to provide a unique way of testing drugs aimed at modifying Gl physiology, and could become a standard for measuring the effect of Gl old and new treatments.
20. Provide self-monitoring capabilities, regardless of disease status. The "quantified self' movement is prompting people of all kinds to monitor their own physiology, regardless of underlying disease. "Gut feelings" are common and have, quite literally, visceral significance. The availability of a wireless, convenient, low profile, continuous abdominal monitoring device will be appealing to anyone curious about their own Gl physiology. The systems 10, 100 can be used to fill a growing demand to provide self-monitoring Gl functions.

In addition to these indications in humans, the systems 10, 100 can be used to provide vital information in veterinary medicine, ranching, and dairy farming. For example, cattle often suffer from rumen sickness, which can kill cattle and severely undermine profits. However, early rumen abnormalities are marked by abnormal digestion and mixing; this can be monitored through acoustics. The systems 10, 100 can be used to predict which cattle are getting worse, and which are not. Early interventions may save the lives of ailing cattle and maximize profits. Similarly, the performance of race horses depends heavily on their Gl health. Understanding and monitoring Gl function in race horses may help to maximize success. The systems 10, 100 may also provide a novel and important way for owners and handlers to monitor the Gl health of horses and other animals otherwise incapable of expressing their digestive health.

In short, virtually every Gl patient may potentially benefit from systems 10, 100 at some point in their diagnostic or therapeutic course. Moreover, the systems 10, 100 can be used for applications beyond Gl patients, and include patients with cardiovascular diseases (e.g. aortic aneurysm) and patients without a defined Gl illness simply interested in monitoring their own physiology. The systems 10, 100 may also have particular applicability in animals. The systems 10, 100 can be configured to be inexpensive, disposable, and widely available, and thus have applicability in multiple markets quickly, provide irreplaceable clinical data, offer a novel way for patients and providers to track disease between scheduled visits, offer the general public a way to quantify their own physiology.

The systems 10, 100 can be configured to include low profile "stickers" (not shown) that adhere to the anterior abdominal wall. One sticker would be placed in the right lower quadrant over the region of the ileocecal valve, the other in the left lower quadrant over the sigmoid colon; other configurations may be possible as needed. The stickers would continuously and non-invasively monitor the same range of gastrointestinal and abdominal wall functions as the belt used in FIG. 1 through FIG 4B.

Data acquired by the systems 10, 100 for each of these applications includes acoustic signals acquired by a network of acoustic sensors worn at the abdomen (and in some case at other anatomical sites). Broadband acoustic signals over the range of at least from dc and to over 100 kHz is acquired. In addition to these signals, abdominal girth will be measured at multiple points along with other physiological variables including, but not limited to, elecrtomyography (EMG) heart rate, respiratory rate and effort, blood pressure, multiple points of core temperature, blood oxygen saturation and others. In addition, patient reporting regarding patient sensation will be acquired along with time and history of nutrition.

Table 1 below presents which data would be acquired for key clinical indications, how the data would be correlated with diagnosing and/or treating a condition, and what diagnosis or treatment protocols would be employed on the basis of data recording and interpretation according to our invention.

Embodiments of the present invention may be described with reference to flowchart illustrations of methods and systems according to embodiments of the invention, and/or algorithms, formulae, or other computational depictions, which may also be implemented as computer program products. In this regard, each block or step of a flowchart, and combinations of blocks (and/or steps) in a flowchart, algorithm, formula, or computational depiction can be implemented by various means, such as hardware, firmware, and/or software including one or more computer program instructions embodied in computer-readable program code logic. As will be appreciated, any such computer program instructions may be loaded onto a computer, including without limitation a general purpose computer or special purpose computer, or other programmable processing apparatus to produce a machine, such that the computer program instructions which execute on the computer or other programmable processing apparatus create means for implementing the functions specified in the block(s) of the flowchart(s).

Accordingly, blocks of the flowcharts, algorithms, formulae, or computational depictions support combinations of means for performing the specified functions, combinations of steps for performing the specified functions, and computer program instructions, such as embodied in computer-readable program code logic means, for performing the specified functions. It will also be understood that each block of the flowchart illustrations, algorithms, formulae, or computational depictions and combinations thereof described herein, can be implemented by special purpose hardware-based computer systems which perform the specified functions or steps, or combinations of special purpose hardware and computer-readable program code logic means.

Furthermore, these computer program instructions, such as embodied in computer-readable program code logic, may also be stored in a computer-readable memory that can direct a computer or other programmable processing apparatus to function in a particular manner, such that the instructions stored in the computer-readable memory produce an article of manufacture including instruction means which implement the function specified in the block(s) of the flowchart(s). The computer program instructions may also be loaded onto a computer or other programmable processing apparatus to cause a series of operational steps to be performed on the computer or other programmable processing apparatus to produce a computer-implemented process such that the instructions which execute on the computer or other programmable processing apparatus provide steps for implementing the functions specified in the block(s) of the flowchart(s), algorithm(s), formula(e), or computational depiction(s).

From the discussion above it will be appreciated that the invention can be embodied in various ways, including the following:
1. An abdominal monitoring system, comprising: a belt configured to be positioned around the waist of a user; one or more sensors coupled to the belt; wherein the belt is configured to support the one or more sensors such that the one or more sensors are positioned adjacent the abdominal wall of the user; and wherein the sensors are configured to receive signals from the abdominal wall; and a controller coupled to the one or more sensors; said controller configured for acquiring data relating to the signals.
2. A system as in any of the previous embodiments, wherein the one or more sensors comprise acoustic sensors configured to receive acoustic signals from the abdominal wall.
3. A system as in any of the previous embodiments, wherein the acoustic sensors comprise digital stethoscopes transducers.
4. A system as in any of the previous embodiments, wherein the acoustic sensors are configured to receive real-time acoustic signals relating to one or more of: gastrointestinal and abdominal wall functions, motility emanating from the gastrointestinal tract and heart rate via abdominal arterial pulse.
5. A system as in any of the previous embodiments, wherein the one or more sensors comprise electromyography sensors to measure contractions of the anterior abdominal musculature.
6. A system as in any of the previous embodiments, wherein the one or more sensors comprise motion sensors to infer breathing rate and other forms of abdominal movement.
7. A system as in any of the previous embodiments, wherein the one or more sensors comprise galvanic skin conductance sensors for evaluation of emotional or physical stress.
8. A system as in any of the previous embodiments, wherein the one or more sensors comprise global positioning sensors to track movement and location within the abdomen.
9. A system as in any of the previous embodiments: wherein said belt comprises an inner inelastic belt having a first end and a second end configured to overlap with the first end when positioned around the abdomen of the user; wherein the first end and second end comprises an array of capacitance measuring electrodes; and wherein the electrodes are configured to measure displacement of the first end with respect to the second end to measure changes in abdominal circumference over time.
10. A system as in any of the previous embodiments, further comprising: programming executable on said processor for: acquiring data relating to the signals; generating an acoustic feature set for each of the one or more sensors; inputting the acoustic feature set to a classifier to produce an output computation corresponding to an inference for a subject condition of the abdomen.
11. A system as in any of the previous embodiments, wherein the acoustic feature set comprises one or more of: amplitude, primary frequency components, time of occurrence of signals and coincidence of signals.
12. A system as in any of the previous embodiments, wherein the classifier comprises a Bayesian classifier.
13. A system as in any of the previous embodiments, wherein the classifier is modified according to training data comprising a library of signals obtained from trials on subjects of a known state.
14. An abdominal monitoring system, comprising: one or more acoustic sensors configured to be positioned adjacent a user's abdominal wall to receive signals from the abdominal wall; and a processor coupled to the one or more acoustic sensors; programming executable on the processor for: acquiring data relating to the signals; generating an acoustic feature set for each of the one or more sensors; and inputting the acoustic feature set to a classifier to produce an output computation corresponding to an inference for a subject condition of the abdomen.
15. A system as in any of the previous embodiments, wherein the acoustic sensors comprise digital stethoscopes transducers.
16. A system as in any of the previous embodiments, wherein the acoustic sensors are configured to receive real-time acoustic signals relating to one or more of: gastrointestinal and abdominal wall functions, motility emanating from the gastrointestinal tract and heart rate via abdominal arterial pulse.
17. A system as in any of the previous embodiments, wherein the acoustic feature set comprises one or more of: amplitude, primary frequency components, time of occurrence of signals and coincidence of signals.
18. A system as in any of the previous embodiments, wherein the classifier comprises a Bayesian classifier.
19. A system as in any of the previous embodiments, wherein the classifier is modified according to training data comprising a library of signals obtained from trials on subjects of a known state.
20. A system as in any of the previous embodiments, further comprising: a belt configured to be positioned around the waist of the user; wherein the belt is configured to support the one or more acoustic sensors such that the one or more sensors are positioned adjacent the abdominal wall of the user.
21. A system as in any of the previous embodiments, further comprising: a wireless transceiver coupled to the one or more acoustic sensors for transferring the acquired sensor data to a remote server.
22. A system as in any of the previous embodiments, wherein the remote server comprises a data fusion module for identification, classification, and notification of abdominal physiological events.
23. A method for monitoring the abdomen of a patient, comprising:
   positioning one or more acoustic sensors configured to be positioned adjacent a user's abdominal wall to receive signals from the abdominal wall; acquiring data relating to the signals; generating an acoustic feature set for each of the one or more sensors; and inputting the acoustic feature set to a classifier to produce an output computation corresponding to an inference for a subject condition of the abdomen.
24. A method as in any of the previous embodiments, wherein the acoustic sensors comprise digital stethoscopes transducers.
25. A method as in any of the previous embodiments, wherein the acoustic sensors are configured to receive real-time acoustic signals relating to one or more of: gastrointestinal and abdominal wall functions, motility emanating from the gastrointestinal tract and heart rate via abdominal arterial pulse.
26. A method as in any of the previous embodiments, wherein the acoustic feature set comprises one or more of: amplitude, primary frequency components, time of occurrence of signals and coincidence of signals.
27. A method as in any of the previous embodiments, wherein the classifier comprises a Bayesian classifier.
28. A method as in any of the previous embodiments, wherein the classifier is modified according to training data comprising a library of signals obtained from trials on subjects of a known state.
29. A method as in any of the previous embodiments, further comprising: transferring the acquired sensor data to a remote server.

Although the description above contains many details, these should not be construed as limiting the scope of the invention but as merely providing illustrations of some of the presently preferred embodiments of this invention. Therefore, it will be appreciated that the scope of the present invention fully encompasses other embodiments which may become obvious to those skilled in the art, and that the scope of the present invention is accordingly to be limited by nothing other than the appended claims, in which reference to an element in the singular is not intended to mean "one and only one" unless explicitly so stated, but rather "one or more." All structural, chemical, and functional equivalents to the elements of the above-described preferred embodiment that are known to those of ordinary skill in the art are expressly incorporated herein by reference and are intended to be encompassed by the present claims. Moreover, it is not necessary for a device or method to address each and every problem sought to be solved by the present invention, for it to be encompassed by the present claims. Furthermore, no element, component, or method step in the present disclosure is intended to be dedicated to the public regardless of whether the element, component, or method step is explicitly recited in the claims. No claim element herein is to be construed under the provisions of 35 U.S.C. 112, sixth paragraph, unless the element is expressly recited using the phrase "means for."

**Table 1. Summary of Key Clinical Indications**

| Condition | Data Acquired | Clinically Relevant Classifications | Treatment Protocols of Relevance |
|---|---|---|---|
| **Postoperative Ileus** | Acoustics Girth | Stage of intestinal recovery, including ileus vs. no ileus, and ability to accept oral intake vs. not prepared for oral intake. | If evidence of postoperative ileus and poor recovery, then no feeding. If no evidence of ileus or signs of recovering ileus, then advance diet to expedite timely discharge. Provides earlier and more accurate classification of post-operative ileus status. |
| **Bowel obstruction** | Acoustics Girth | No bowel obstruction vs. partial bowel obstruction vs. complete bowel obstruction. | If evidence of partial or complete bowel obstruction, then no feeding, careful monitoring, abdominal imaging, and possibly abdominal surgery on a timely basis. Provides earlier identification of bowel obstruction and earlier medical and/or surgical intervention. |
| **Intensive Care Status** | Acoustics Girth EMG | "Acute abdomen" vs. "benign abdomen." Especially relevant among non-communicative patients, common in the ICU. | If evidence of abdominal expansion, hyperdynamic bowel sounds, or abdominal wall tension suggesting pain, evaluate for evidence of acute abdominal infection, inflammatory process, or obstruction. Provides earlier identification of evolving intra-abdominal processes, and trigger earlier diagnosis through directed physical exam and/or imaging. |
| **Ascites** | Girth | Static and dynamic status of ascites (mild, moderate, severe, worsening vs. improving). | If evidence of worsening ascites evidenced through expanding abdominal girth, then increase diuretic dose and reduce salt in diet. The system may allow earlier identification of poorly-controlled ascites among outpatients. |
| **Narcotic Bowel** | Acoustics Girth | Overdose vs. appropriate dose of narcotics. | If evidence of ileus in setting of narcotics, reduce dose and titrate to level that maintains maximal 24-hour Gl acoustics while controlling pain. The system may allow more rationale and evidence-based dosing of narcotics while minimizing resource-intensive Gl side effects. |
| **IBD** | Acoustics Girth EMG | Bowel obstruction from strictures vs. no obstruction, Hypermotility and/or diarrhea vs. normotility and normal stool frequency. | If evidence of evolving bowel obstruction in at-risk outpatients, then arrange earlier provider visit and/or abdominal imaging. If evidence of increasing motility and bowel movement frequency, then increase medication doses or start new targeted therapies. Provides more timely and accurate identification of worsening clinical status among outpatients with ulcerative colitis and Crohn's. |
| **Abdominal Bloating** | Acoustics Girth EMG | Bloating from gas mishandling vs. functional bloating with abdominal wall relaxation. | If evidence of functional bloating, then behavioral therapy; if evidence of gas mishandling, then antibiotic and dietary therapy. |
| **Diarrhea/Fecal Incontinence** | Acoustics | Anorectal dysfunction vs. meal related vs. volume related mechanisms. | If evidence of anorectal dysfunction, then directed therapy for anorectum; if volume, then medical therapies. |
| **Constipation** | Acoustics Girth | Slow vs. normal transit vs. pelvic dyssynergia mechanisms. | If slow transit, then medications to increase motility. If norma transit, consider centrally acting therapies. If dyssynergia, then behavioral therapies. |
| **Irritable Bowel Syndrome (IBS)** | Acoustics Girth EMG Galvanic | Central vs. peripheral mechanism of IBS symptoms. | If central, then centrally-acting therapies and behavioral modification. If peripheral, then peripheral medical therapies for motility, bacteria, etc. Improves monitoring and categorizing patients with IBS. |

## Claims

1. An abdominal monitoring system, comprising:
one or more acoustic sensors configured to be positioned adjacent a user's abdominal wall to receive signals from the abdominal wall; and
a processor coupled to the one or more acoustic sensors;
programming executable on the processor for:
acquiring data relating to the signals;
generating an acoustic feature set for each of the one or more sensors; and
inputting the acoustic feature set to a classifier to produce an output computation corresponding to an inference for a subject condition of the abdomen.

2. A system as recited in claim 1, further comprising:
a belt configured to be positioned around the waist of the user;
wherein the belt is configured to support the one or more acoustic sensors such that the one or more sensors are positioned adjacent the abdominal wall of the user.

3. A system as recited in claim 1, further comprising:
a wireless transceiver coupled to the one or more acoustic sensors for transferring the acquired sensor data to a remote server.

4. A system as recited in claim 8, wherein the remote server comprises a data fusion module for identification, classification, and notification of abdominal physiological events.

5. A method for monitoring the abdomen of a patient, comprising:
positioning one or more acoustic sensors configured to be positioned adjacent a user's abdominal wall to receive signals from the abdominal wall;
acquiring data relating to the signals;
generating an acoustic feature set for each of the one or more sensors; and inputting the acoustic feature set to a classifier to produce an output computation corresponding to an inference for a subject condition of the abdomen.

6. A method as recited in claim 5 or a system as recited in claim 1, wherein the acoustic sensors comprise digital stethoscopes transducers.

7. A method as recited in claim 5 or a system as recited in claim 1, wherein the acoustic sensors are configured to receive real-time acoustic signals relating to one or more of:
gastrointestinal and abdominal wall functions, motility emanating from the gastrointestinal tract and heart rate via abdominal arterial pulse.

8. A method as recited in claim 5 or a system as recited in claim 1, wherein the acoustic feature set comprises one or more of: amplitude, primary frequency components, time of occurrence of signals and coincidence of signals.

9. A method as recited in claim 5 or a system as recited in claim 1, wherein the classifier comprises a Bayesian classifier.

10. A method as recited in claim5 or a system as recited in claim 1, wherein the classifier is modified according to training data comprising a library of signals obtained from trials on subjects of a known state.

11. A method as recited in claim 5, further comprising:
transferring the acquired sensor data to a remote server.

12. A method as recited in claim 6, further comprising:
analyzing the acquired data for identification, classification, and notification of abdominal physiological events.
